# EUROPÄISCHE PATENTSCHRIFT

(11) **EP 1 599 459 B1**
(45) Veröffentlichungstag und Bekanntmachung des Hinweises auf die Patenterteilung: **03.10.2007**
(21) Anmeldenummer: 04710797.4
(22) Anmeldetag: 13.02.2004
(51) Int. Cl.: C07D 317/42

(54) **VERFAHREN ZUR HERSTELLUNG VON 4-FLUOR-1,3-DIOXOLAN-2-ON**
METHOD FOR PRODUCING 4-FLUORO-1,3-DIOXOLAN-2-ONE
PROCEDE DE PRODUCTION DE 4-FLUORO-1,3-DIOXOLAN-2-ONE

(30) Priorität: 26.02.2003 DE 10308149
(43) Veröffentlichungstag der Anmeldung: 30.11.2005
(73) Patentinhaber: Solvay Fluor GmbH, 30173 Hannover (DE)
(72) Erfinder: BÖSE, Olaf, 30167 Hannover (DE); SEFFER, Dirk, 31535 Neustadt (DE); PETERKORD, Katja, 30449 Hannover (DE)
(74) Vertreter: Jacques, Philippe
(86) Internationale Anmeldenummer: PCT/EP2004/001345
(87) Internationale Veröffentlichungsnummer: WO 2004/076439

(56) Entgegenhaltungen:
- DATABASE WPI Section Ch, Week 200112 Derwent Publications Ltd., London, GB; Class B03, AN 2001-106329 XP002284419 & JP 2000 309583 A (KANTO DENKA KOGYO K.K.), 7. November 2000 (2000-11-07) in der Anmeldung erwähnt & PATENT ABSTRACTS OF JAPAN vol. 2000, no. 14, 5. März 2001 (2001-03-05) & JP 2000 309583 A (KANTO DENKA KOGYO CO LTD), 7. November 2000 (2000-11-07)
- HASEGAWA, MASARU ET AL: "Electroorganic synthesis under solvent-free conditions. Highly regioselective anodic monofluorination of cyclic ethers, lactones, and a cyclic carbonate" TETRAHEDRON LETTERS (2002), 43(8), 1503-1505, XP004334975

## Beschreibung

Die Erfindung betrifft ein Verfahren zur Herstellung von 4-Fluor-1,3-dioxolan-2-on mit hoher Selektivität. 4-Fluor-1,3-dioxolan-2-on kann durch stöchiometrische Umsetzung von Chlorethylencarbonat mit Kaliumfluorid hergestellt werden. Die Reaktionsdauer beträgt hier etwa 24 Stunden. JP 2000 309583 beschreibt ein Verfahren zur Herstellung von 4-Fluor-1,3-dioxolan-2-on durch Umsetzung von Ethylencarbonat mit Fluor. Da Direktfluorierungsreaktionen sehr stark exotherm sind, wird das Ethylencarbonat in organischen Lösungsmitteln gelöst und das Fluorgas oder eine fluorhaltige Gasmischung in das Ethylencarbonat eingeleitet. Die Reaktionstemperatur liegt hier im Bereich von 20 bis 100°C. Direktfluorierungen sind stark exotherme Reaktionen, es muss also Sorge getragen werden, die Reaktionstemperatur im beherrschbaren Rahmen zu halten. Gemäß JP 2000 309583 wird die eingeleitete Gasphase gekühlt.

Die Aufgabe der Erfindung besteht darin ein modifiziertes Verfahren zur Herstellung von 4-Fluor-1,3-dioxolan-2-on bereitzustellen. Das Verfahren soll sich durch eine hohe Selektivität und eine gute Temperaturführung auszeichnen.

Erfindungsgemäß wird Ethylencarbonat mit 3 bis 20 Gew.-% 4-Fluor-1,3-dioxolan-2-on (bezogen auf die Ethylencarbonatmenge) versetzt. Die Lösung kann zunächst auf ca. 35°C erwärmt werden, um das Ethylencarbonat aufzulösen. In die Reaktionslösung wird Fluorgas oder ein Gemisch, das Fluor in einem Inertgas enthält, eingeleitet. Fluor wird unterstöchiometrisch bezogen auf vorhandenes Ethylencarbonat eingesetzt, um die Bildung von Nebenkomponenten einzuschränken, z.B. werden Fluormengen im Bereich von 60 bis 95 Mol %, vorzugsweise 70 bis 95 Mol % (bezogen auf vorhandenes Ethylencarbonat) eingesetzt. Während der gesamten Reaktionsdauer wird die Reaktionslösung auf eine Temperatur von 15 bis 45°C gekühlt. Nur zu Beginn der Fluorierung liegt die Temperatur der Lösung kurzzeitig oberhalb des Schmelzpunktes von Ethylencarbonat (37-39 °C). Über 90 % der Fluorierungszeit ist die Temperatur der Lösung unterhalb.des Schmelzpunktes von Ethylencarbonat. In einer Ausführungsform beträgt die Temperatur der Lösung 20 bis 35°C. Die hierbei erhaltene Reaktionslösung wird nach sorgfältiger Neutralisation der entstandenen Fluorwasserstoffsäure mehrfach destillativ unter Vakuum gereinigt. Ethylencarbonathaltige Fraktionen können als Ausgangsmaterial für das erfindungsgemäße Verfahren verwendet werden. Dadurch kann das nicht umgesetzte Ethylencarbonat im Prozess rezykliert werden.

Die für das erfindungsgemäße Verfahren benötigte erste Menge an 4-Fluor-1,3-dioxolan-2-on kann nach einem bekannten Verfahren hergestellt werden.

Es wurde gefunden, dass der Schmelzpunkt des Reaktionsgemisches durch Zugabe von 4-Fluor-1,3-dioxolan-2-on gesenkt wird. Dadurch ist es möglich, die Reaktionspartner bereits unterhalb der Schmelztemperatur des Ethylencarbonats miteinander reagieren zu lassen.

Es wurde weiterhin gefunden, dass durch Zugabe des 4-Fluor-1,3-dioxolan-2-on nicht nur die Temperatur des Reaktionsgemisches gesenkt werden kann, sondern dadurch auch gleichzeitig die Reaktivität gemindert wird.

Die Selektivität der Reaktion wird dadurch erheblich verbessert, was sich darin zeigt, dass weniger Fluor eingesetzt werden muss und die Ausbeute an zu Produkt umgesetztem Fluor steigt. Die Bildung von Nebenprodukten wird dadurch ebenfalls eingeschränkt. So konnte durch Halbierung der eingesetzten Fluormenge die Ausbeute gemäß den bekannten Verfahren erzielt werden.

Ein weiterer Vorteil des erfindungsgemäßen Verfahrens besteht darin, dass kein zusätzliches separates inertes Lösungsmittel benötigt wird, das nach der Synthese des 4-Fluor-1,3-dioxolan-2-ons wieder abgetrennt werden muss. Die Aufarbeitung der erhaltene Reaktionslösung wird dadurch vereinfacht.

Die nachfolgenden Beispiele sollen die Erfindung erläutern jedoch nicht einschränken.

### Beispiel 1

Ethylencarbonat besitzt einen Schmelzpunkt von 37-39 °C, während 4-Fluor-1,3-dioxolan-2-on einen Schmelzpunkt von etwa 17 °C zeigt. Durch die Zugabe von 4-Fluor-1,3-dioxolan-2-on zu Ethylencarbonat kann dessen Schmelzpunkt gesenkt werden. So zeigt eine Mischung von 87% Ethylencarbonat und 13 % 4-Fluor-1,3-dioxolan-2-on einen Schmelzpunkt von etwa 20 °C.

### Beispiel 2

2000 g Ethylencarbonat wurde bei Raumtemperatur in einem PFA-Behälter vorgelegt. 616 g einer 4-Fluor-1,3-dioxolan-2-on haltigen Lösung, die Ethylencarbonat enthält, wurden hinzugeführt. Die gesamte Reaktionslösung enthielt 95,03 Gew.-% Ethylencarbonat (2483,4 g; 28,22 mol) und 4,88 Gew.-% 4-Fluor-1,3-dioxolan-2-on (129,3 g; 1,22 mol).

Diese Suspension wurde auf 35°C erwärmt, dabei löste sich das vorgelegte Ethylencarbonat vollständig.

Danach wurde ein Fluor/Stickstoff-Gasgemisch (5:95 Vol%) über ein Tauchrohr mit PTFE-Fritte eingeleitet.

Insgesamt wurden 25,23 mol Fluor (berechnet auf 100 % Fluor) eingeleitet.

Zu Beginn der Fluorierung liegt die Temperatur der Lösung kurzzeitig oberhalb des Schmelzpunktes von Ethylencarbonat. Über 90 % der Fluorierungszeit ist die Temperatur der Lösung unterhalb des Schmelzpunktes von Ethylencarbonat, vorzugsweise im Bereich von 30 bis 35°C.

Nach Beendigung der Reaktion wurde die Reaktionslösung gereinigt, indem ihr zunächst Aceton zugesetzt wurde. Danach wurde sie mit Kaliumhydrogencarbonat unter Rühren neutralisiert. Die erhaltene Suspension wurde über eine Nutsche abgesaugt und der Rückstand nochmals mit Aceton gewaschen. Die erhaltene Lösung wurde mehrmals unter Vakuum destilliert.

Entsprechend wurden 1839g (17,34 mol) 4-Fluor-1,3-dioxolan-2-on erhalten. Nach Abzug der ursprünglich enthalten Mengen an 4-Fluor-1,3-dioxolan-2-on ergibt sich für 4-Fluor-1,3-dioxolan-2-on eine Ausbeute von 63,9 % bezogen auf die eingesetzte Fluormenge.

## Patentansprüche

1. Verfahren zur Herstellung von 4-Fluor-1,3-dioxolan-2-on durch Umsetzung von Ethylencarbonat mit elementarem Fluor, **dadurch gekennzeichnet, dass** in einem Reaktionsbehälter Ethylencarbonat mit 3-20 Gew.-% 4-Fluor-1,3-dioxolan-2-on (bezogen auf Ethylencarbonat) als Lösungsmittel für Ethylencarbonat vermischt wird, und in die Lösung bei einer Temperatur von 15 bis 45°C Fluorgas oder ein Gemisch, das Fluor in einem Inertgas enthält, eingeleitet wird.

2. Verfahren zur Herstellung von 4-Fluor-1,3-dioxolan-2-on nach Anspruch 1, **dadurch gekennzeichnet, dass** dem Ethylencarbonat 4 bis 14 Gew.-% 4-Fluor-1,3-dioxolan-2-on (bezogen auf Ethylencarbonat) als Lösungsmittel für Ethylencarbonat hinzugefügt werden.

3. Verfahren nach Anspruch 1 bis 2, **dadurch gekennzeichnet, dass** der Reaktion keine weiteren Lösungsmittel zugesetzt werden.

4. Verfahren nach Anspruch 1 bis 3 **dadurch gekennzeichnet, dass** die Reaktion unterhalb des Schmelzpunktes von Ethylencarbonat durchgeführt wird.

5. Verfahren nach Anspruch 1 bis 4 **dadurch gekennzeichnet, dass** Fluor in unterstöchiometrischen Mengen, vorzugsweise 60 bis 95 Mol %, bezogen auf vorhandenes Ethylencarbonat eingesetzt wird, um die Bildung von Nebenkomponenten einzuschränken.

6. Verfahren nach Anspruch 1 bis 5 **dadurch gekennzeichnet, dass** die nicht umgesetzten Mengen an Ethylencarbonat rezykliert werden.

## Claims

1. A process for the preparation of 4-fluoro-1,3-dioxolan-2-one by reaction of ethylene carbonate with elemental fluorine, **characterised in that** ethylene carbonate is mixed in a reaction vessel with 3-20 % by weight 4-fluoro-1,3-dioxolan-2-one (relative to ethylene carbonate) as solvent for ethylene carbonate, and fluorine gas or a mixture containing fluorine in an inert gas is introduced into the solution at a temperature of 15 to 45°C.

2. A process for the preparation of 4-fluoro-1,3-dioxolan-2-one according to Claim 1, **characterised in that** 4 to 14 % by weight 4-fluoro-1,3-dioxolan-2-one (relative to ethylene carbonate) is added to the ethylene carbonate as solvent for ethylene carbonate.

3. A process according to Claim 1 to 2, **characterised in that** no further solvents are added to the reaction.

4. A process according to Claim 1 to 3, **characterised in that** the reaction is carried out below the melting point of ethylene carbonate.

5. A process according to Claims 1 to 4, **characterised in that** fluorine is used in hypostoichiometric amounts, preferably 60 to 95 mol %, relative to ethylene carbonate present, in order to restrict the formation of secondary components.

6. A process according to Claim 1 to 5, **characterised in that** the unreacted amounts of ethylene carbonate are recycled.

## Revendications

1. Procédé pour la préparation de la 4-fluoro-1,3-dioxolan-2-one par mise en réaction de carbonate d'éthylène avec du fluor élémentaire, **caractérisé en ce qu'**on mélange, dans un récipient de réaction, du carbonate d'éthylène avec de la 4-fluoro-1,3-dioxolan-2-one à concurrence de 3 à 20 % en poids (rapportés au carbonate d'éthylène) comme solvant pour le carbonate d'éthylène, et on introduit dans la solution, à une température de 15 à 45 °C, du fluor gazeux ou un mélange qui contient du fluor dans un gaz inerte.

2. Procédé pour la préparation de la 4-fluoro-1,3-dioxolan-2-one selon la revendication 1, **caractérisé en ce qu'**on ajoute au carbonate d'éthylène, de la 4-fluoro-1,3-dioxolan-2-one à concurrence de 4 à 14 % en poids (rapportés au carbonate d'éthylène) à titre de solvant pour le carbonate d'éthylène.

3. Procédé selon les revendications 1 à 2, **caractérisé en ce qu'**on n'ajoute à la réaction aucun solvant supplémentaire.

4. Procédé selon les revendications 1 à 3, **caractérisé en ce qu'**on effectue la réaction en dessous du point de fusion du carbonate d'éthylène.

5. Procédé selon les revendications 1 à 4, **caractérisé en ce qu'**on met en oeuvre du fluor dans des quantités sous-stoechiométriques, de préférence à concurrence de 60 à 95 moles % rapportés au carbonate d'éthylène présent pour limiter la formation de sous-produits.

6. Procédé selon les revendications 1 à 5, **caractérisé en ce qu'**on recycle les quantités de carbonate d'éthylène n'ayant pas réagi.
